Europäisches Patentamt

(19) European Patent Office

Office européen des brevets

(11) Publication number: **0 048 357**
**A1**

## (12) EUROPEAN PATENT APPLICATION

(21) Application number: **81106832.9**

(22) Date of filing: **01.09.81**

(51) Int. Cl.³: **G 01 N 33/54**
**A 61 K 39/395**

(30) Priority: **12.09.80 SE 8006424**

(43) Date of publication of application:
**31.03.82 Bulletin 82/13**

(84) Designated Contracting States:
**BE DE FR GB NL SE**

(71) Applicant: **LA JOLLA CANCER RESEARCH**
**FOUNDATION**
**2945 Science Park Road**
**La Jolla California 92037(US)**

(72) Inventor: **Engvall, Eva**
**262 Via Del Cerrito**
**Olivenhain, Ca 92024(US)**

(72) Inventor: **Ruoslahti, Erkki**
**262 Via Del Cerrito**
**Olivenhain, Ca 92024(US)**

(72) Inventor: **Uotila, Marjatta**
**6336 Rutgers**
**Houston Texas 77030(US)**

(74) Representative: **Kraus, Walter, Dr. et al,**
**Patentanwälte Dres. Kraus & Weisert Irmgardstrasse 15**
**D-8000 München 71(DE)**

(54) Method for the determination of an antigen in solution.

(57) An improvement in methods for the determination of an antigen (I) in solution, in which determination said antigen (I) is reacted with an antibody (II), which is directed against the antigen (I), and with an antibody (III), which is directed against the antigen (I) and is labelled with an analytically indicatable atom or group and is soluble in the liquid in the presence of which the determination is carried out, to the formation of a conjugate comprising said antigen (I) and said antibodies (II) and (III), which conjugate is insoluble or is made insoluble, whereafter the analytically indicatable atom or group is determined in the insoluble or insolubilized conjugate and/or in the solution. The improvement comprises using as antibody (II) and antibody (III) in said determination antibodies which are monoclonal and react with roomly spaced determinants of the antigen (I).

EP 0 048 357 A1

Croydon Printing Company Ltd.

## METHOD FOR THE DETERMINATION OF AN ANTIGEN IN SOLUTION

The present invention relates to a method for the determination of an antigen in solution.

More particularly, the present invention relates to a method for the determination of an antigen (I) in solution, in which determination said antigen (I) is reacted with an antibody (II), which is directed against the antigen (I) and with an antibody (III), which is directed against the antigen (I) and is labelled with an analytically indicatable atom or group and is soluble in the liquid in the presence of which the determination is carried out, to the formation of a conjugate comprising said antigen (I) and said antibodies (II) and (III), which conjugate is insoluble or is made insoluble, whereafter the analytically indicatable atom or group is determined in the insoluble or insolubilized conjugate and/or in the solution.

Usually and preferably said conjugate is insoluble or is made insoluble by said antibody (II) being insoluble or being made insoluble by bonding to a water-insoluble solid phase. Thus, the antibody (II) may be insoluble before the formation of the conjugate by being bound to a water-insoluble solid phase or the antibody (II) is made insoluble after the formation of the conjugate by bonding to a water-insoluble solid phase whereby the conjugate is made insoluble also. The water-insoluble phase may be a water-insoluble polymer. The antibody (II) may be bound to the water-insoluble polymer by covalent bonds. Other methods of making the conjugate insoluble are also possible.

A great number of assay methods involving biospecific affinity reactions is known in which methods a first immunochemical reactant is reacted with a second immunochemical reactant exhibiting biospecific affinity to said first reactant and then a third immunochemical reactant, which exhibits bio-

2

0048357

specific affinity to the first or the second reactant (i.e.
is an immunochemical counterpart to the first or the second
reactant) is reacted with its counterpart to the formation of
a conjugate comprising said first, second and third reactants,
one of said reactants being labelled with an analytically
indicatable atom or group of atoms.

The term "immunochemical reactant" as used in this context
includes immunoglobulins (including modified immunoglobulins,
e.g. aggregated, and fragments e.g. Fab- or Fc-fragments),
preferably antibodies, and antigens and haptens.

In order to enable the determination of the analytically
indicatable atom or group in the conjugate a separation of said
conjugate and free labelled reactant (i.e. labelled reactant
which is not bound in the conjugate) is carried out.

A common way of achieving such a separation is to use as
one of the reactants (but not the labelled reactant) a reactant
which is bound to an insoluble polymer resulting in the conjugate formed by the biospecific reactions being attached to the
insoluble polymer so that said conjugate can be separated from
the free labelled reactant which remains in solution. According to one group of such methods a water-insoluble polymer
material is used to which an antibody or an antigen is bound.
Thus, for instance, British Patent Specifications Nos. 1 192 784,
1 248 764 and 1 248 765 and also Biochem. Biophys. Acta 130
(1966) page 257 and Radioimmunoassay Methods (Editors: K. E.
Kirkham and W. M. Hunter, Churchil Livingstone, London 1971)
e.g. the article "Solid Phase Antigen Antibody Systems" by
L. Wide, pp 405-412, disclose the use of a water-insoluble
polymer material having an antibody or an antigen bound thereto
by bonds of covalent nature. Further, US Patent Specification
No. 3 646 346 discloses an immunochemical assay method using
antibodies adsorbed to the inside of a test tube of a plastics
material.

A recent way of achieving separation of the conjugate is
disclosed in Swedish Patent Application No. 8003732-8.
According to this reference the conjugate formed by the different reactants is soluble in water but after its formation it is bound covalently to an insoluble carrier or to an insolubilizable carrier which latter carrier is made insoluble after the covalent bonding of the conjugate thereto.

Labelling of an antigen or an antibody with an analytically indicatable atom or group, for instance a radioactive atom or group, a fluorescent, luminescent or chromophoric group or an enzymatically active group, an enzyme inhibitor group or a co-enzyme group, is well-known nowadays and well established techniques for this labelling are generally known. In this connection it is known that the labelling group or atom can be bound directly to the antigen or antibody or that a bridge may be introduced between the antigen or antibody and the label.

According to another aspect assay methods involving biospecific affinity reactions may be grouped into two types of methods, viz, competitive methods and "sandwich" methods.

In competitive methods an unlabelled reactant in a sample and corresponding labelled reactant are reacted with the immunochemical counterpart to said reactants under competition.

In sandwich methods, a first reactant, bound to an insoluble or insolubilizable carrier, is reacted with a reactant to be determined in a sample which latter reactant then is reacted with a labelled immunochemical counterpart thereto, which counterpart may be labelled first reactant.

When determining an antigen in a sample a sandwich type method is often preferred since such a method does not require the use of labelled antigen which in certain cases is difficult to procure. However, the sandwich assay places particularly stringent requirements on the specificity of the antibody used and an assay procedure with multiple incubation and washing steps is usually required.

According to the present invention an improved sandwich assay method of the type set forth in the introductory part above is provided, which method is characterized in using as antibody (II) and antibody (III) in said determination antibodies which are monoclonal and react with roomly spaced (sterically spaced) determinants of the antigen (I).

Thus, at the formation of the conjugate the monoclonal antibody (II) can bind to a site on the antigen (I) and the labelled monoclonal antibody (III) can bind to another site on the antigen (I), whereby the two sites located on the antigen (I) are spaced from each other so that antibody (II) and antibody (III) can both at the same time occur bound to antigen (I) without constituting a sterical hindrance one to the other. The two binding sites on the antigen (I) can for example be located on different sides of the antigen (I), e.g. on different sides of a protein molecule.

When compared to the prior art use of the antibodies of a common antiserum, which comprises a population of different antibodies having varying specificities and affinities and which antibodies can be directed against different sites (determinants) on the antigen (I), the use of monoclonal antibodies according to the invention affords a conciderable progress and enables more accurate analysis and also analytical procedures which have not been possible before.

Thus, the method according to the invention is an improvement in clinical diagnosis.

According to the preferred embodiment of the method according to the invention said antibodies (II) and (III) are monoclonal antibodies which are directed against structurally different determinants of the antigen (I). This embodiment makes a contemporaneous or substantially contemporaneous addition of the two antibodies possible, thus eliminating one incubation and washing procedure as compared with the prior art technique. Thus, the antibodies (II) and (III) are preferably added to the sample to be tested for antigen (I) at least substantially contemporaneously when practising this embodiment.

According to another embodiment of the method according to the present invention, which embodiment is to be applied to the determination of an antigen exhibiting two equal determinants which are roomly spaced as is the case of e.g. fibronectin, antibody (II) is bound to an insoluble carrier (e.g. an insoluble polymer) and antibodies (II) and (III) are monoclonal antibodies directed against said determinants. When practising this embodiment the antigen (I) is reacted with antibody (II) and then with antibody (III). Due to sterical obstacles caused by the solid carrier only one of the two equal determinants of the antigen (I) is reacted with antibody (II), thus leaving the other determinant free to react with antibody (III).

The preparation of monoclonal antibodies is described in literature; vide for instance G. Köhler and C. Milstein Nature 256, 495 (1975) Galfre et al., Nature 266 (1977) 550, Oi et al., Current topics in Microbiology and Immunology 81 (1978) 115-129 and the article "Immunoglobulin-Producing Hybrid Cell Lines" by V. T. Oi and L. A. Herzenberg in Selective Methods in Cellular Immunology (edited by Mishell B.B. & Shiigi S. M.) in Press 1980.

Briefly the preparation of monoclonal antibodies may be carried out for example as follows:

A plasma cell, secreting predefined antibody, from the spleen of the immunized mouse, is fused with a myeloma cell capable of vigorous and continuous growth. Selection of myeloma: spleen cell hybrids are accomplished by culturing the fusion mixture in hypoxanthine-aminopterin-thymidine medium (HAT medium). Supernates of the surviving hybrid cell cultures are then tested for antibody activity. An aliquot of cells from antibody-producing cultures is grown and prepared for freezing, while another aliquot is employed in cloning of the hybrids. In the latter procedure the cells are grown at limiting dilutions to derive cloned cell lines. Clones that secret the desired antibody are then expanded, and several aliquots frozen while others are used for large-scale antibody production. The resulting antibodies are then purified and characterized.

Monoclonal antibodies to be used as antibody (II) in the method according to the invention can be bound to an insoluble or insolubilizable carrier using the prior art technique disclosed for the bonding of immunochemical reactants to such carriers. The bonding to the carrier material may be carried out before or after the reaction of antibody (II) with the antigen (I) or even after the formation of the conjugate comprising the antigen (I) and the antibodies (II) and (III).

Bonding by covalent bonds may be carried out before the reaction with the antigen (I) by means of well-known coupling agents such as glutardialdehyde, cyanogen bromide, etc. whereas the bonding technique disclosed in Swedish Patent Application No. 8003732-8 may be used for covalent coupling of antibody (II) before as well as after the reaction with antigen (I) and the formation of the above mentioned conjugate.

When applying last-mentioned technique a carrier and an antibody (II) are used which exhibit one type each of reactive groups, said reactive groups of the two reactants being capable of reacting with each other to the formation of a covalent bond between the carrier and said antibody. For instance, one of the two reactants (i.e. the carrier or the antibody) may exhibit pyridyl-disulphide groups and the other SH-groups which groups are able to react with each other in a thiol-disulphide exchange reaction to the formation of a disulphide bridge between the carrier and the antibody. This combination of reactive groups may also be used in case of a water-soluble carrier, which is made insoluble after the covalent bonding of antibody (II) to the carrier has been carried out. In this case a water-soluble polymer containing pyridyldisulphide groups may for example be used as the carrier and an antibody (II) may be used which exhibits HS-groups which react with the pyridyl-disulphide group of the carrier in a thiol-disulphide exchange reaction to the coupling together of the antibody and the carrier over a -S-S-bridge. The number of pyridyldisulphide groups is selected to be in considerable excess as compared to the number of HS-groups of the antibodies. After the coupling together of the carrier and the antibody, part of the residual pyridyldisulphide groups of the carrier are transferred into HS-group by treatment with a reducing agent such as dithio-threitol. A polymerisation of the carrier molecules to the formation of an insoluble product by reaction between the HS-groups thus formed and residual pyridyl-disulphide groups will then take place.

Labelling of the monoclonal antibody to be used as antibody (III) with at least one analytically indicatable atom or group is carried out using the conventional technique for the labelling of antibodies.

Like in the prior art methods the determination is preferably carried out in the presence of an aqueous liquid, e.g. a buffer solution of a suitable pH and suitable ion strength. The antigen may be in solution in said liquid, including colloidal solution.

The invention is further illustrated by the following examples.

Example 1

A)    Preparation of monoclonal antibodies

Antibodies to human alphafetoprotein (AFP) were produced by hybridization of mouse myeloma cells with spleen cells from mice immunized with AFP. The hybridization and subsequent culture and cloning of the hybrids were performed essentially according to Oi and Herzenberg in Selective Methods in Cellular Immunologi, edited by Mishell B.B. & Shiigi S.M. in Press.

Briefly BALB/C Dub mice (Flow Laboratories, Dublin, Va., USA) were immunized by subcutaneous injections of 50 µg of purified AFP emulsified in complete Freunds adjuvant. The injections were repeated 2-3 times at 3-4 week interval. A final injection of AFP was given intraperitoneally and 3-5 days later the spleens of the mice were removed for hybridization.

The myeloma cell line (reference: The Journal of Immunology, Vol 123, No 4, 1979, pages 1548 - 1550) was used for hybridization. $10^8$ spleen cells were mixed with 1-3 x $10^7$ myeloma cells in Dulbecco's medium (from Flow Laboratories, Inglewood, Ca., USA). The cells were spun down to form a tight pellet and all the supernatant was removed. 1 ml 50% (w/v) polyethylene glycol (Mw = 1500) was added slowly with gently mixing of the cells. After one minute the cell suspension was diluted slowly with 5 ml of Dulbecco's medium (from Flow Laboratories, Inglewood, Ca., USA). The cells were spun down, washed and distributed into two 96 well plates in Dulbecco's medium containing 10% (w/v) fetal calf serum 100 IU/ml penicillin and 100 µg/ml streptomycin. The plates were placed into 7% $CO_2$ incubation at $37^{O}C$. Half of the culture medium in each well was replaced by fresh medium containing hypoxanthine-aminopterin-thymidin (HAT) $10^{-4}$ hypoxanthine, $4 \times 10^{-7}$ M aminopterin and $1.6 \times 10^{-5}$ M thymidin on days 1, 2 and 3 and every 3 days thereafter for 2 weeks. The cultures were tested for anti AFP activity using the enzyme-linked immunosorbent assay, ELISA (Eva Engvall and Peter Perlmann, J. Immunol. 109, 129-135, 1972). The positive cultures detected by this assay were cloned repeatedly using the limiting dilution method (Oi and Herzenberg, 1980) and then grown in culture or as ascites tumours in mice.

B)    Coating of microtitre plates

The monoclonal antihuman AFP antibodies were diluted in 0.1 M sodium carbonate buffer pH 9.5 to a concentration of 5 µg/ml. Wells in non-treated polystyrene microtitre plates (Titertek from Flow Laboratories, Inglewood, Ca., USA) were coated with 200 µl of the diluted antibodies overnight at room temperature. The plates were washed three times with 0.9% by weight sodium chloride solution containing 0.05% by volume polyoxyethylene sorbitan monolaurate (Tween®20).

C)    Preparation of enzyme-labelled antihuman AFP antibodies

Mouse monoclonal anti AFP antibodies were conjugated with horse radish peroxidase according to the following procedure:
5 mg of horse radish peroxidase (peroxidase Type VI from Sigma Chemical Company, St. Louis, USA) were dissolved in 100 µl of phosphate buffered saline pH 7.2 with 1.25% (w/v) glutardialdehyde. The solution was incubated overnight at room temperature. 0.9 ml 0.1 M sodium carbonate buffer pH 9.2 was added and the mixture was dialysed against 0.1M $NaHCO_3$ for 8 hours. 2.5 mg of mouse anti AFP antibodies were added and the mixture was incubated overnight at room temperature. Possibly remaining reactive groups were blocked by the addition of 0.1 ml of 0.2 M lysine. The conjugate formed can be stored in 50% (w/v) glycerol.

D)    Determination of AFP

The analysis was performed in the wells of microtitre plates pre-coated according to B) above.

0.05 ml of phosphate buffered saline with 0.05% by volume of Tween®20 and 1% (w/v) steer serum pH 7.2 was added to all wells.

0.1 ml of the sample to be tested was added to one of the wells (A).

0.1 ml of standard solutions containing 100, 50, 25, 12.5, 6.25, 3.1 and 1.5 µg AFP /l was added to respectively wells B, C, D, E, F, G, H and I.

0.05 ml of the solution containing the enzyme-labelled antibody (from C) above) diluted 100 times in phosphate buffered saline pH 7.2 was added to each well and the mixture was incubated for 2 h at +37°C.

The wells were then washed 3 times with 0.9% by weight sodium chloride containing 0.05% by volume Tween®20.

0.2 ml of a substrate comprising 0.4 mg/ml o-phenylendiamine in 0.1 m citrate-phosphate buffer pH 5.0 containing 0.01% w/v $H_2O_2$ was added for 30 minutes at room temperature.

The colour development was read spectrophotometrically at 450 nm. (If the colour development is stopped by the addition of 4M $H_2SO_4$ the absorbance should be measured at 492 nm.)

The results when using two different monoclonal antibodies (i.e. antibodies directed against roomly spaced determinants) were:

| AFP concentration µg/l | Absorbance at 450 nm |
|---|---|
| 100 | 1.6 |
| 50 | 0.82 |
| 25 | 0.44 |
| 12.5 | 0.25 |
| 6.25 | 0.13 |
| 3.1 | 0.07 |
| 1.5 | 0.03 |

The results when the same monoclonal antibody was coated to the wells as was used for the enzyme conjugate were:

| AFP concentration µg/l | Absorbance at 450 nm |
| --- | --- |
| 100 | 0.02 |
| 50 | 0.02 |
| 25 | 0.02 |
| 12.5 | 0.02 |
| 6.25 | 0.02 |
| 3.1 | 0.02 |
| 1.5 | 0.02 |

Example 2

A)    Preparation of monoclonal antibodies

Mouse monoclonal anti AFP antibodies are prepared according to the procedure described in Example 1A.

B)    Preparation of paper discs with covelently bound antibodies

The antibodies of A) above are coupled to BrCN activated paper discs according to the procedure described by M. Ceska and U. Lundkvist in Immunochemistry Vol. 9, No. 10, pp 1021-1030.

· C)    Preparation of $^{125}$I-labelled AFP antibody

Monoclonal anti AFP antibodies prepared according to Example 1A are labelled with $^{125}$I according to the method described by Hunter and Greenwood (Nature, Vol. 194, 1962, p. 495).

D)    Determination of AFP

The assay is performed essentially as was described by M. Ceska and U. Lundkvist in Immunochemistry, Vol. 9, No. 10,

BAD ORIGINAL

pp. 1021-1030 for IgE, except for that all the components are added simultaneously. The incubation time is 3 hours at room temperature. The tubes with the washed discs are placed in a counter for estimating gamma radiation. The number of "counts" per time unit for the standards are entered on a counts dose diagram on a lin log scale from which the amount of AFP in the unknown test samples can be calculated.

Example 3

A)   Preparation of monoclonal antibodies

Mouse monoclonal antihuman fibronectin antibodies were prepared according to the procedure described in Example 1 A) for mouse monoclonal antiAFP antibodies.

B)   Coating of microtitre plates

The antibodies of A) above were diluted in 0.1 M sodium carbonate buffer pH 9.5 to a concentration of 3 µg/ml.

Wells of microtitre plates were coated in the manner described in Example 1 B).

C)   Preparation of enzyme-labelled anti human fibronectin antibodies

Mouse monoclonal antihuman fibronectin antibodies (a-HFN) were conjugated with alkaline phosphatase according to the following procedure.

300 µl (1.5 mg) alkaline phosphatase (phosphatase Type VII 5 mg/ml in $(NH_4)_2SO_4$, from Sigma Chemical Company, St. Louis, USA) and 100 µl a-HFN (from A) above) in phosphate buffered saline pH 7.2 were dialysed under high pressure in cold overnight against phosphate buffered saline pH 7.2 to get rid of $(NH_4)_2SO_4$. 25% (w/v) glutardialdehyde was added to a final concentration of 0.2% (w/v). The mixture was incubated for 3 h at room temperature and then dialysed against phosphate buffered saline pH 7.2 overnight.

D) <u>Determination of HFN</u>

The analysis was performed in the wells of microtitre plates precoated according to B) above.

0.2 ml of a sample to be tested was added to one of the wells (A).

0.2 ml of a standard solution containing 4000, 800, 160, 32 and 6.4 µg HFN/1 was added to respectively wells B, C, D, E, F and G.

After incubation for 3 h at +37$^{\circ}$C the wells were washed three times with 0.9% by weight NaCl containing 0.05% by volume Tween®20.

0.2 ml of the solution from C) above containing the enzyme-labelled antibody was added to each of the wells and then incubation was performed for 3 h at +37$^{\circ}$C, whereafter the wells were washed three times with 0.9% by weight NaCl containing 0.05% by volume Tween®20.

0.2 ml of a substrate comprising 1 mg/ml p-nitrophenyl phosphate (from Sigma Chemical Company, St. Louis, USA) in 1.0 M diethanolamine-HCl buffer pH 9.8 containing 1 mM $MgCl_2$ was added and 30 minutes later 0.5 ml of 0.5N NaOH was added.

The colour development was read spectrophotometrically at 405 nm.

The results when the same monoclonal antibody was coated to the wells as was used for the enzyme conjugate were:

| HFN concentration µg/1 | Absorbance at 405 nm |
| --- | --- |
| 4000 | 2.0 |
| 800 | 1.1 |
| 160 | 0.52 |
| 32 | 0.25 |
| 6.4 | 0.12 |

BAD ORIGINAL

CLAIMS

1.    Method for the determination of an antigen (I) in solution, in which determination said antigen (I) is reacted with an antibody (II), which is directed against the antigen (I), and with an antibody (III), which is directed against the antigen (I) and is labelled with an analytically indicatable atom or group and is soluble in the liquid in the presence of which the determination is carried out, to the formation of a conjugate comprising said antigen (I) and said antibodies (II) and (III), which conjugate is insoluble or is made insoluble, whereafter the analytically indicatable atom or group is determined in the insoluble or insolubilized conjugate and/or in the solution, c h a r a c t e r i z e d  in using as antibody (II) and antibody (III) in said determination antibodies which are monoclonal and react with roomly spaced determinants of the antigen (I).

2.    Method according to claim 1, c h a r a c t e r i z e d  in using as antibody (II) an antibody (II), which is bound to a water-insoluble solid phase.

3.    Method according to claim 2, c h a r a c t e r i z e d  in that the antibody (II) is bound by covalent bonds to a water-insoluble polymer.

4.    Method according to claims 1-3, c h a r a c t e r i z e d  in using as antibodies (II) and (III) monoclonal antibodies which are directed against structurally different determinants of the antigen (I).

5.    Method according to claim 4, c h a r a c t e r i z e d  in that the antibodies (II) and (III) are added to a sample at least substantially contemporaneously.

6.    Method according to claims 1-3, c h a r a c t e r i z e d  in that the antigen (I) to be determined exhibits two equal determinants which are roomly spaced, that antibody (II) is bound to an insoluble carrier and that antibodies (II) and (III) are monoclonal antibodies directed against said determinants.

| | DOCUMENTS CONSIDERED TO BE RELEVANT | | CLASSIFICATION OF THE APPLICATION (Int. Cl. 3) |
|---|---|---|---|
| Category | Citation of document with indication, where appropriate, of relevant passages | Relevant to claim | |
| P/X | CHEMICAL ABSTRACTS, vol. 94, no. 23, 8th June, 1981, page 469, abstract 190054g, COLUMBUS, OHIO (US) M. UOTILA et al.: "Two-site sandwich enzyme immunoassay with monoclonal antibodies to human alpha-fetoprotein" & J. Immunol. Methods 1981, 42(1), 11-15 <br><br> * the whole abstract * | 1-6 | G 01 N 33/54 <br> A 61 K 39/395 |
| P/X | GASTROENTEROLOGY, vol. 79, no. 5, part 2, November 1980, J.R. WANDS et al.: "Immunodiagnosis of hepatitis B by high affinity monoclonal anti-HB antibodies", page 1063, column 1 <br><br> * the entire article * | 1-3 | **TECHNICAL FIELDS SEARCHED (Int.Cl. 3)** <br><br> G 01 N 33/54 <br> 33/56 <br> 33/58 <br> 33/68 <br> A 61 K 39/395 |
| P | GASTROENTEROLOGY, vol. 79, no. 5, part 2, November 1980, J.R. WANDS et al.: "Identification of epitopes on HBsAg polypeptides by analysis with monoclonal anti-HBs antibodies", page 1063, column 1 <br><br> * the entire article * | 2-4 | |
| | GASTROENTEROLOGY, vol. 77, no. 5, 1979, J.R. WANDS et al.: "Production and characterization of monoclonal antibodies to hepatitis B surface antigen (HBsAg) by cellular hybridization", page A46, column 1 <br><br> * the entire article * <br><br> ./.. | 1,2,4 | **CATEGORY OF CITED DOCUMENTS** <br><br> X: particularly relevant if taken alone <br> Y: particularly relevant if combined with another document of the same category <br> A: technological background <br> O: non-written disclosure <br> P: intermediate document <br> T: theory or principle underlying the invention <br> E: earlier patent document, but published on, or after the filing date <br> D: document cited in the application <br> L: document cited for other reasons <br><br> &: member of the same patent family, corresponding document |

| | The present search report has been drawn up for all claims | | |
|---|---|---|---|
| Place of search <br> The Hague | Date of completion of the search <br> 13.01.1982 | Examiner <br> GRIFFITH | |

EPO Form 1503.1 06.78

European Patent
Office

**EUROPEAN SEARCH REPORT**

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| P | NATURE, vol. 290, no. 5806, 9th April 1981, BUCKS (GB) D.S. SECHER: "Immunoradiometric assay of human leukocyte interferon using monoclonal antibody", pages 501-503 <br><br> * the entire document * | 1,2 | |
| | CHEMICAL ABSTRACTS, vol. 94, no. 25, 22nd June, 1981, page 115, abstract 203339f, COLUMBUS, OHIO (US) F. BUCHEGGER et al.: "Use of monoclonal anti-CEA antibodies in immunoadsorbent columns and solid-phase radioimmunoassay" & Protides Biol Fluids 1980, 28th, 511-515 <br><br> * the entire document * | 1 | TECHNICAL FIELDS SEARCHED (Int. Cl.³) |
| | JOURNAL OF IMMUNOLOGICAL METHODS, vol. 39, no. 4, 1980, R. JEFFERIS et al.: "Quantitation of human total IgG, kappa IgG and lambda IgG in serum using mono-clonal antibodies", pages 355-362 <br><br> * the entire article * | 4 | |